(19) Europäisches Patentamt
European Patent Office
Office européen des brevets

(11) **EP 4 327 721 A1**

(12) **EUROPEAN PATENT APPLICATION**
published in accordance with Art. 153(4) EPC

(43) Date of publication:
**28.02.2024 Bulletin 2024/09**

(21) Application number: **21937937.7**

(22) Date of filing: **23.04.2021**

(51) International Patent Classification (IPC):
***A61B 1/045*** (2006.01)

(52) Cooperative Patent Classification (CPC):
**A61B 1/045**

(86) International application number:
**PCT/JP2021/016473**

(87) International publication number:
**WO 2022/224446 (27.10.2022 Gazette 2022/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **NEC Corporation
108-8001 Tokyo (JP)**

(72) Inventors:
• **WATANABE, Kazuhiro
Tokyo 108-8001 (JP)**
• **SAIKOU, Masahiro
Tokyo 108-8001 (JP)**
• **EBIHARA, Akinori
Tokyo 108-8001 (JP)**
• **MIYAGAWA, Taiki
Tokyo 108-8001 (JP)**

(74) Representative: **Betten & Resch
Patent- und Rechtsanwälte PartGmbB
Maximiliansplatz 14
80333 München (DE)**

(54) **IMAGE PROCESSING DEVICE, IMAGE PROCESSING METHOD, AND STORAGE MEDIUM**

(57)    An image processing device 1X includes an acquisition means 30X, a score calculation means 31X, and a classification means 34X. The acquisition means 30X acquires each captured image which captures an examination target by an imaging unit provided on an endoscope. The score calculation means 31X calculates a score concerning a likelihood of an existence of a point of interest in captured images based on a plurality of captured images which are in a time series. The classification means 34X classifies the plurality of captured images based on the score.

FIG. 11

EP 4 327 721 A1

Processed by Luminess, 75001 PARIS (FR)

**Description**

TECHNICAL FIELD

The present disclosure relates to a technical field of an image processing device, an image processing method, and a storage medium for processing each image to be acquired during an endoscopic examination.

BACKGROUND ART

An endoscopy system which displays images of a lumen of an organ have been known for many years. For example, Patent Document 1 discloses a learning method for a learning model which outputs information concerning a lesion site included in captured image data in a case of inputting image data generated by an imaging device. Moreover, Patent Document 2 discloses a classification method for classifying sequential data by a method applying a sequential probability ratio test (SPRT: Sequential Probability Ratio Test).

PRECEDING TECHNICAL REFERENCES

PATENT DOCUMENT

Patent Document 1: International Publication WO2020/003607
Patent Document 2: Internationally published WO2020/194497

SUMMARY

PROBLEM TO BE SOLVED BY THE INVENTION

In a case of detecting a lesion site from images taken during an endoscopic examination, the lesion site is detected by individually analyzing images obtained by an endoscope one by one. However, with this method, there is a possibility that a presence of the lesion site and the like which are difficult to distinguish from a single image may not be properly detected.
It is one object of the present disclosure to provide an image processing apparatus, an image processing method, and a storage medium which are capable of appropriately detecting an area of interest such as a lesion site in an endoscopic examination.

MEANS FOR SOLVING THE PROBLEM

According to an example aspect of the present disclosure, there is provided an image processing device including:

an acquisition means configured to acquire each captured image which captures an examination target by an imaging unit provided on an endoscope;
a score calculation means configured to calculate a score concerning a likelihood of an existence of a point of interest in captured images based on a plurality of captured images which are in a time series; and
a classification means configured to classify the plurality of captured images based on the score.

According to another example aspect of the present disclosure, there is provided an image processing method performed by a computer, the image processing method including:

acquiring each captured image which captures an examination target by an imaging unit provided on an endoscope;
calculating a score concerning a likelihood of an existence of a point of interest in captured images based on a plurality of captured images which are in a time series; and
classifying the plurality of captured images based on the score.

According to a further example aspect of the present disclosure, there is provided a storage medium storing a program, the program causing a computer to perform a process including:

acquiring each captured image which captures an examination target by an imaging unit provided on an endoscope;
calculating a score concerning a likelihood of an existence of a point of interest in captured images based on a plurality of captured images which are in a time series; and
classifying the plurality of captured images based on the score.

EFFECT OF THE INVENTION

According to the present disclosure, it is possible to appropriately detect an area of interest such as a lesion site in an endoscopic examination.

BRIEF DESCRIPTION OF THE DRAWINGS

[FIG. 1] FIG. 1 illustrates a schematic configuration of an endoscopy system.
[FIG. 2] FIG. 2 illustrates a hardware configuration of an image processing device.
[FIG. 3] FIG. 3 illustrates a functional block diagram of the image processing device.
[FIG. 4] FIG. 4 illustrates a graph representing a transition of a classification score calculated based on a first manner.
[FIG. 5] FIG. 5 illustrates a graph representing a transition of a classification score calculated based on a second manner.
[FIG. 6] FIG. 6 illustrates a first display example of a display screen displayed by a display device in an endoscopic examination.
[FIG. 7] FIG. 7 illustrates a second display example of the display screen displayed by the display device in the endoscopic examination.
[FIG. 8] FIG. 8 illustrates a third display example of the display screen displayed by the display device in the endoscopic examination.
[FIG.9] FIG. 9 illustrates an example of a flowchart for explaining a process procedure based on the first manner.
[FIG. 10] FIG. 10 illustrates an example of a flowchart for explaining a process procedure based on the second manner.
[FIG. 11] FIG. 11 illustrates a block diagram of an image processing device in a second example embodiment.
[FIG. 12] FIG. 12 illustrates an example of a flowchart of a process executed by the image processing device in the second example embodiment.

EXAMPLE EMBODIMENTS

In the following, example embodiments will be described with reference to the accompanying drawings.

<First Example Embodiment>

(1) System Configuration

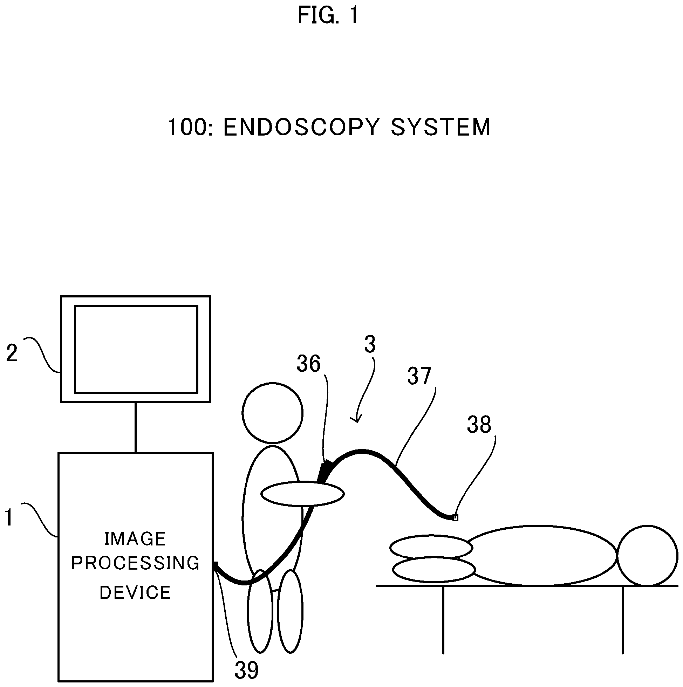

FIG. 1 illustrates a schematic configuration of an endoscopy system 100. As illustrated in FIG. 1, the endoscopy system 100 is a system for presenting a suspected lesion site (lesion site) to an examiner such as a physician who performs an examination or treatment using an endoscope, and mainly includes an image processing device 1, a display device 2, and an endoscopic scope 3 which is connected to the image processing device 1.
The image processing device 1 acquires each image (also referred to as a "captured image Ia") captured by the endoscopic scope 3 in time series from the endoscopic scope 3, and displays a screen based on the captured image Ia on the display device 2. The captured image Ia is an image captured by a predetermined frame period during at least one of an insertion process or an ejection process of the endoscopic scope 3 to an examinee. In the present example embodiment, by analyzing the captured image Ia, the image processing device 1 detects the presence of the lesion site in the captured image Ia, and displays information concerning a detection result on the display device 2.
The display device 2 is a display or the like for performing a predetermined display based on a display signal supplied from the image processing device 1.
The endoscopic scope 3 mainly includes an operation portion 36 for the examiner to perform a predetermined input, a shaft 37 which has flexibility and is inserted into an organ to be photographed by the examinee, a tip portion 38 with an imaging unit such as a built-in ultra-compact image sensor, and a connection portion 39 to connect to the image processing device 1.
The configuration of the endoscopy system 100 depicted in FIG.1 is an example, and may be modified in various manners. For instance, the image processing device 1 may be formed integrally with the display device 2. In another example, the image processing device 1 may be formed by a plurality of devices.

Hereafter, as a representative example, a process in an endoscopy of a large intestine will be described; however, an examination target is not limited to a colon but may also be an esophagus or a stomach. Moreover, the endoscopes covered in this disclosure include, for example, pharyngoscopy, bronchoscopy, upper gastrointestinal endoscopy, duodenoscopy, small bowel endoscopy, colonoscopy, capsule endoscopy, thoracoscopy, laparoscopy, cystoscopy, biliary endoscopy, arthroscopy, spine endoscopy, vascular endoscopy, and epidural endoscopy. In addition, the pathology of the lesion site to be detected in the present disclosure is exemplified as follows (a) to (f)

(a) Head and neck: pharyngeal cancer, malignant lymphoma, papilloma
(b) Esophagus: esophageal cancer, esophagitis, hiatal hernia, Barrett's esophagus, esophageal varices, esophageal achalasia, esophageal submucosal tumor, benign esophageal tumor
(c) Stomach: gastric cancer, gastritis, gastric ulcer, gastric polyp, gastric tumor
(d) Duodenum: duodenal cancer, duodenal ulcer, duodenitis, duodenal tumor, duodenal lymphoma
(e) Small intestine: small intestine cancer, small intestine neoplastic disease, small intestine inflammatory disease, small intestine vascular disease
(f) Large intestine: colorectal cancer, colorectal neoplastic disease, colorectal inflammatory disease, colorectal polyps, colorectal polyposis, Crohn's disease, colitis, intestinal tuberculosis, hemorrhoids

(2) Hardware Configuration

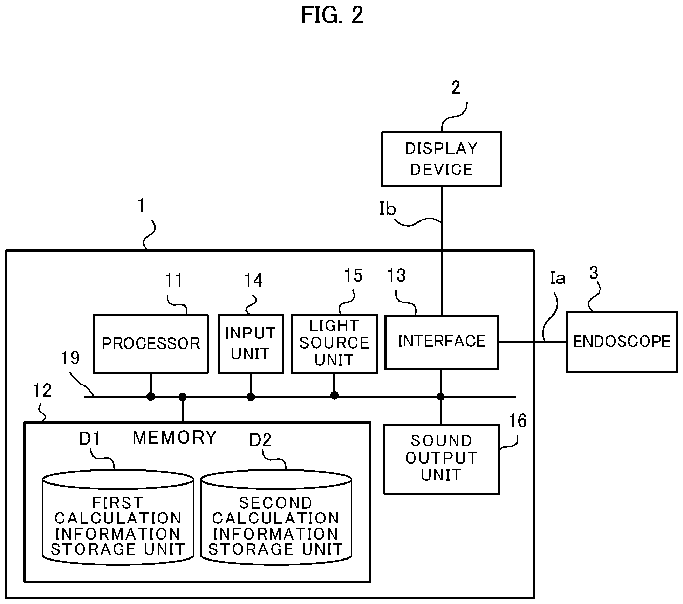

FIG. 2 illustrates a hardware configuration of the image processing device 1. The image processing device 1 mainly includes a processor 11, a memory 12, an interface 13, an input unit 14, a light source unit 15, and a sound output unit 16. These elements are mutually connected via a data bus 19.

The processor 11 executes a predetermined process by executing a program or the like stored in the memory 12. The processor 11 is a processor such as a CPU (Central Processing Unit), a GPU (Graphics Processing Unit), and a TPU (Tensor Processing Unit). The processor 11 may be formed by a plurality of processors. The processor 11 is an example of a computer.

The memory 12 is formed by any of various volatile memories used as working memories such as a RAM (Random Access Memory) and a ROM (Read Only Memory) and a non-volatile memory for storing information necessary for processing the image processing device 1. The memory 12 may include an external storage device such as a hard disk which is connected to or embedded in the image processing device 1, and may include a storage medium such as a removable flash memory. The memory 12 stores a program for executing a process according to the present example embodiment by the image processing device 1.

Moreover, the memory 12 functionally includes a first calculation information storage unit D1 for storing the first calculation information and a second calculation information storage unit D2 for storing second calculation information. The first calculation information and the second calculation information are information used when the image processing device 1 classifies the presence or absence of the lesion site and information indicating a result of the calculation concerning a classification, and details thereof will be described later. In addition, the memory 12 stores various parameters necessary for calculating a score of the classification regarding the existence or absence of the lesion site. At least a portion of the information stored in the memory 12 may be stored in an external device other than the image processing device 1. In this case, the above-described external device may be one or more server devices capable of data communication with the image processing device 1 through a communication network or the like or through direct communication.

The interface 13 performs interface operation between the image processing device 1 and the external device. For instance, the interface 13 supplies display information 'Ib' generated by the processor 11 to the display device 2. Also, the interface 13 supplies light or the like generated by the light source unit 15 to the endoscopic scope 3. The interface 13 also provides an electronic signal to the processor 11 representing the captured image Ia which is supplied from the endoscopic scope 3. The interface 13 may be a communication interface such as a network adapter for wired or wireless communication with the external device, or may be a hardware interface compliant with a USB (Universal Serial Bus), a SATA (Serial AT Attachment), or the like.

The input unit 14 generates an input signal based on an operation by the examiner. The input unit 14 is, for instance, a button, a touch panel, a remote controller, a voice input device, or the like. The light source 15 generates light to be supplied to the tip portion 38 of the endoscopic scope 3. Moreover, the light source unit 15 may also incorporate a pump or the like for delivering water or air to be supplied to the endoscope scope 3. The sound output unit 16 outputs sound based on the control of the processor 11.

(3) Detection Process of Lesion Site

Next, a detection process of the lesion site by the image processing device 1 will be described. Schematically, the image processing device 1 detects the lesion site based on a variable number of the captured images Ia in time series. Accordingly, the image processing device 1 accurately detects the lesion site even for the lesion site which is difficult to discriminate from a single image, and presents the detection result. Note that the lesion site which is difficult to discriminate from the single image may be, for instance, a non-cancerous area or a flat cancerous area in Barrett's esophagus.

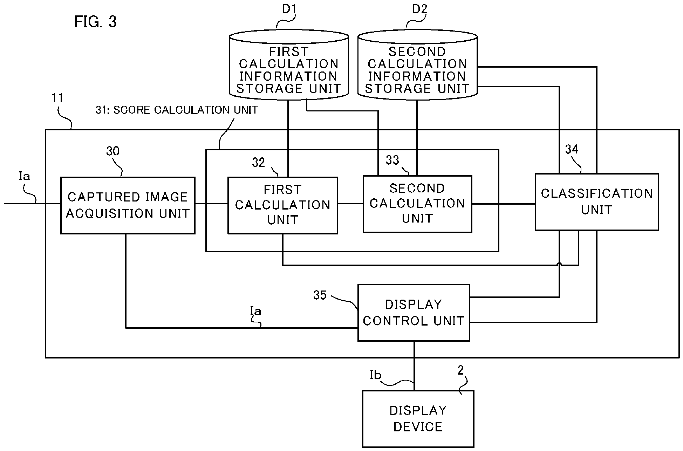

FIG. 3 is a functional block diagram of the image processing device 1. As illustrated in FIG. 3, the processor 11 of the image processing device 1 functionally includes a captured image acquisition unit 30, a score calculation unit 31, a classification unit 34, and a display control unit 35. In FIG. 3, blocks for sending and receiving data are connected by solid lines, but a combination of blocks for sending and receiving data is not limited to that in FIG. 3. The same applies to the drawings for other functional blocks described below.

The captured image acquisition unit 30 acquires the captured images Ia captured by the endoscopic scope 3 through the interface 13 at predetermined intervals in accordance with the frame period of the endoscopic scope 3. Next, the captured image acquisition unit 30 supplies the acquired captured images Ia to each of the score calculation unit 31 and the display control unit 35.

The score calculation unit 31 is for use in the classification of the presence or absence of the lesion site, and calculates a score indicating a likelihood that the lesion site is present (also referred to as a "classification score". In the present example embodiment, the score calculation unit 31 determines the classification score based on a likelihood ratio concerning the captured images Ia in the time series. Here, the "likelihood ratio concerning the captured images Ia in the time series" refers to a ratio of the likelihood that the lesion site exists in the captured images Ia in the time series and the likelihood that the lesion site does not exist in the captured images Ia in the time series. In the present example embodiment, as an example, it is assumed that the likelihood ratio increases as the likelihood that the lesion site exists is greater.

The score calculation unit 31 functionally includes a first calculation unit 32 and a second calculation unit 33.

The first calculation unit 32 calculates the likelihood ratio for the latest "N" (N is an integer) captured images Ia for each process time of each predetermined time length, and provides the calculation result to the first calculation unit 32. In this case, for instance, the first calculation unit 32 calculates the likelihood ratio using, for instance, a likelihood ratio calculation model which has been trained to output the likelihood ratio for the N captured images Ia being input upon receiving the N captured images Ia. The likelihood ratio calculation model may be a deep learning model, any other machine learning model or a statistical model. In this case, for instance, in the memory 12, learned parameters of the likelihood ratio calculation model are stored, and the first calculation unit 32 inputs the latest N captured images Ia to the likelihood ratio calculation model configured by referring to the parameters, and acquires the likelihood ratio output by the likelihood ratio calculation model. In a case where the likelihood ratio calculation model is formed by a neural network, various parameters such as a layer structure, a neuron structure in each layer, a number of filters and filter sizes in each layer, and respective weights of elements for each filter are stored in memory 12 in advance.

The likelihood ratio calculation model may include an optional feature extraction unit which extracts features (that is, feature vectors) for each captured image Ia which is input to the likelihood ratio calculation model, and may be separately formed from such the feature extraction unit. In the latter case, the likelihood ratio calculation model becomes a model which is trained to output the likelihood ratio concerning the N captured images Ia when the features of the N captured images Ia extracted by the feature extraction unit are input. Moreover, preferably, the feature extraction unit may be a unit which extracts features representing a relationship of time series data based on any technique for calculating the relationship of the time series data such as a LSTM (Long Short Term Memory).

The first calculation unit 32 stores the likelihood ratio calculated at respective process times and data used by the first calculation unit 32 to calculate the likelihood ratio in the first calculation information storage unit D1 as first calculation information. The "data used to calculate the likelihood ratio" may correspond to the captured images Ia used to calculate the likelihood ratio, and may be features extracted from these captured images Ia.

The second calculation unit 33 calculates the likelihood ratio (also referred to as an "integrated likelihood ratio") integrating the likelihood ratios calculated at a current process time and previous process times, and determines the classification score based on the integrated likelihood ratio. Note that the classification score may be the integrated likelihood ratio itself or may be a function including the integrated likelihood ratio as a variable.

Here, assuming that the current process time is "t" and any captured image or its feature to be processed is "$x_i$" (i=1, ... , t), the integrated likelihood ratio concerning a binary classification between a class "R" including the lesion site and a class "F" in which the captured images Ia do not include the lesion site is expressed by the following equation (1).

[Math 1]

$$
log \left[ \frac{p(x_1, \ldots, x_t | R)}{p(x_1, \ldots, x_t | F)} \right]
$$

$$
= \sum_{s=N+1}^{t} log \left[ \frac{p(R | x_s, \ldots, x_{s-N})}{p(F | x_s, \ldots, x_{s-N})} \right] - \sum_{s=N+2}^{t} log \left[ \frac{p(R | x_{s-1}, \ldots, x_{s-N})}{p(F | x_{s-1}, \ldots, x_{s-N})} \right]
$$

Here, "p" denotes the probability belonging to each class (that is, confidence level 0 to 1). In calculating a term on a right side of the equation (1), it is possible to use the likelihood ratio stored in the first calculation information storage D1 by the first calculation unit 32 as the first calculation information.

In the equation (1), since the current process time t increases with time, the length in time series of the captured images Ia (or their features) used to calculate the integrated likelihood ratio is variable. Accordingly, by using the integrated likelihood ratio based on the equation (1), as a first advantage, the second calculation unit 33 can calculate , as a first advantage, the classification score by considering a variable number of the captured images Ia. In addition, by using the integrated likelihood ratio based on the equation (1), as a second advantage, it is possible to classify time-dependent features, and as a third advantage, it is possible to suitably calculate the classification score which enables the classification with less loss of accuracy even for data which are difficult to discriminate. The second calculation unit 33 stores the integrated likelihood ratio and the classification score calculated at the respective process times as the second calculation information in the second calculation information storage unit D2.

The classification unit 34 performs the classification concerning the presence or absence of the lesion site based on the classification score calculated by the second calculation unit 33. In this case, the classification unit 34 performs the above-described classification using a threshold of the classification score on the presence or absence of the lesion site. In the present example embodiment, as an example, a higher classification score indicates a higher likelihood that the lesion site is present, and the classification unit 34 determines that the lesion site exists in the captured images Ia used to calculate the classification score with respect to the classification score that is higher than a predetermined threshold value (also referred to as a "first threshold value Th1"). On the other hand, with respect to the classification score that is equal to or less than a predetermined threshold value (also referred to as a "second threshold value Th2") which is less than the first threshold value, the classification unit 34 determines that there is no lesion site in the captured images Ia used to calculate the classification score. Each of first threshold Th1 and the second threshold Th2 is, for instance, a fitted value determined based on experiments or the like, are stored in the memory 12 or the like in advance. Next, while the classification score does not reach these thresholds, the classification unit 34 does not perform the determination of the classification, and holds (undetermines) the classification for the captured images Ia of interest. Thereafter, "performing the classification" includes holding the classification as well as determining (deciding) the classification. A specific manner of the classification by the classification unit 34 will be described later. The classification unit 34 supplies a classification result to the display control unit 35.

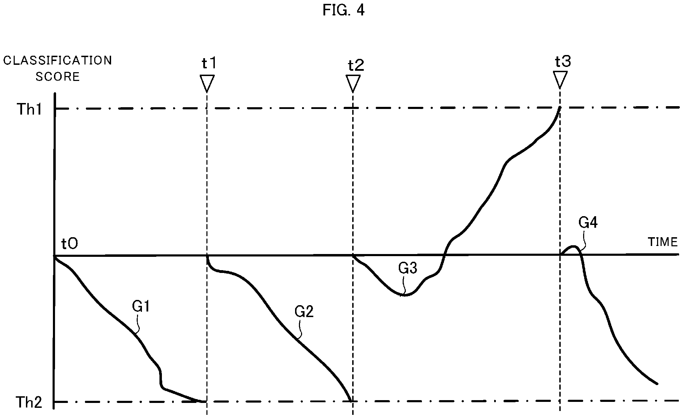

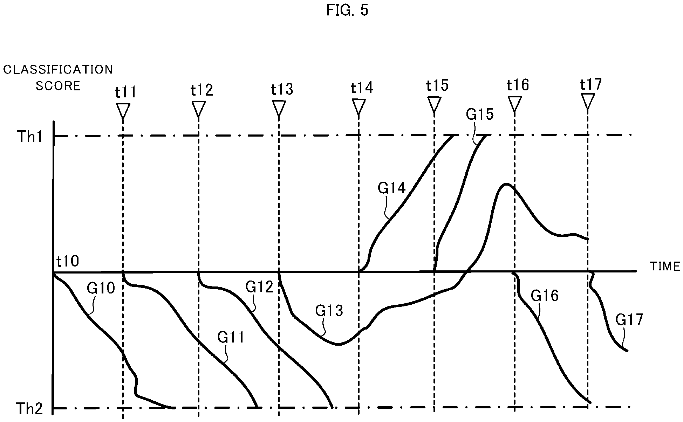

Moreover, when the classification score is equal to or less than the first threshold value Th1 or the second threshold value Th2, the classification unit 34 sends a notification indicating that the classification is determined to the score calculation unit 31. In this case, the first calculation unit 32 and the second calculation unit 33 of the score calculation unit 31 reset (initialize) the process result before the notification and calculate the classification score based on the captured images Ia newly generated from a time of receiving the notification. A specific example of this process will be described in detail with reference to FIG. 4 and FIG. 5.

The display control unit 35 generates display information Ib based on the captured images Ia and the classification result supplied from the classification unit 34, and displays the captured images Ia and information concerning the classification result by the classification unit 34 to be displayed on the display device 2 by supplying the display information Ib to the display device 2 via the interface 13. Moreover, the display control unit 35 may suitably further display information such as information concerning the classification score stored in the second calculation information storage unit D2, on the display unit 2. The display example of the display control unit 35 will be described later.

Here, each component of the captured image acquisition unit 30, the score calculation unit 31, the classification unit 34, and the display control unit 35 can be realized, for instance, by the processor 11 which executes corresponding programs. Moreover, the necessary programs may be recorded on any non-volatile storage medium and installed as necessary to realize respective components. Note that at least some of these components may be realized by any combination of hardware, firmware, software, and the like, without limitation to realization by software with programs. At least some of these components may be realized using user-programmable integrated circuits, such as field-programmable gate arrays (FPGAs) or microcontrollers. In this case, the integrated circuit may be used to realize respective programs corresponding to the above components. Furthermore, at least a portion of the components may be formed by an ASSP (Application Specific Standard Produce), an ASIC (Application Specific Integrated Circuit), or a quantum processor (quantum computer control chip). Thus, each of the components may be realized by various hardware. The above is the same in other example embodiments described later. Furthermore, each of these components may be implemented by the cooperation of a plurality of computers, for instance, using cloud computing technology.

Next, specific manners (a first manner and a second manner) for a calculation process of the classification score will be described. In general, in the first manner, the score calculation unit 31 initializes the classification score by re-setting a point of start (also referred to as a "start point") of the captured images Ia used for calculating the classification score each time the classification score reaches either the first threshold value Th1 or the second threshold value Th2. In the second manner, the score calculation unit 31 sets the start point at each predetermined time interval, and performs calculations of the classification score in parallel for respective start points which have been set.

FIG. 4 illustrates graphs "G1" to "G4" representing a transition of the classification score calculated based on the first manner. In this example, the score calculation unit 31 starts a process from a time "t0" and calculates the classification score based on the captured images Ia acquired by the frame period of the endoscopic scope 3.

Here, in the period from a time t0 to a time t1, since the captured images Ia including the lesion site are not generated, the classification score gradually approaches the second threshold value Th2 as illustrated in the graph G1. Note that, since the classification score has not reached any threshold value until the time t1, the classification unit 34 holds the determination of the classification for the captured images Ia and makes the classification undetermined. In this case, the classification unit 34 may supply information indicating that the classification is undetermined to the display control unit 35.

Then, at the time "t1", the classification score calculated by the score calculation unit 31 based on the captured images Ia obtained from the time t0 to the time t1 decreases to or less than the second threshold value Th2. Therefore, in this case, the classification unit 34 supplies the display control unit 35 with the classification result that the lesion site is not included in the captured images Ia obtained from time t0 to time t1, and supplies the score calculation unit 31 with a notification for re-setting the calculation process of the classification score (that is, to reset the start point).

Then, the score calculation unit 31 resets the calculation process of the classification score based on the notification indicating that the classification is determined from the classification unit 34 at the time t1. Next, the score calculation unit 31 starts the calculation process of the classification score for the captured images Ia obtained after the time t1. After that, as illustrated in the graph G2, at a time "t2", the classification score calculated by the score calculation unit 31 based on the captured images Ia obtained from the time t1 to the time t2 decreases equal to or less than the second threshold value Th2. Accordingly, in this case, the classification unit 34 generates the classification result that the lesion site is not included in the captured images Ia obtained from the time t1 to the time t2. Next, the classification unit 34 supplies the generated classification result to the display control unit 35, and supplies the notification for re-setting the calculation process of the classification score to the score calculation unit 31.

Next, at a time t3, the score calculation unit 31 starts the calculation process of the classification score for the captured images Ia acquired after the time t2 based on the notification which is sent from the classification unit 34 and indicates that the classification has been performed. Here, since the captured images Ia obtained from the time t2 to the time t3 include the lesion site, the classification score calculated by the score calculation unit 31 after the time t2 gradually approaches the first threshold value Th1 as illustrated in the graph G3.

Then, in the time "t3", the classification score calculated by the score calculation unit 31 based on the captured images Ia obtained from the time t2 to the time "t3" increases equal to or greater than the first threshold value Th1. Therefore, in this case, the classification unit 34 generates the classification result indicating that the captured images Ia obtained from the time t2 to the time t3 include the lesion site. Next, the classification unit 34 supplies the generated classification result to the display control unit 35, and supplies the notification for re-setting the calculation process of the classification score to the score calculation unit 31. After that, at the time t3, the score calculation unit 31 starts the calculation process of the classification score for the captured images Ia acquired after the time t2 based on the notification from the classification unit 34 that the classification is determined (see the graph G4).

As described above, in the first manner, the score calculation unit 31 re-sets the calculation process of the classification score when the classification score is equal to or greater than the first threshold value Th1 or lower than the second threshold value Th2 (that is, when the determination result of the classification is made), in consideration of the fact that the target which is captured by the endoscopic scope 3 changes over time. Accordingly, the score calculation unit 31 can suitably eliminate an influence of the determination result of a part to be previously captured, and can calculate the classification score so that the classification score appropriately reaches the first threshold value Th1 for the lesion site that is the target to be captured.

FIG. 5 illustrates graphs "G10" to "G17" representing a transition of a classification score calculated based on the second manner. In this example embodiment, the score calculation unit 31 starts the process from a time "t10" and calculates the classification score based on the captured images Ia acquired by the predetermined frame period. Here, as an example, in a vicinity of the period from a time "t14" to a time "t16", it is assumed that the lesion site is included in the captured images Ia.

In this instance, the score calculation unit 31 sets a start point of the captured images Ia used for the calculation of the classification score by a predetermined time interval from the time t10, and starts a thread (process flow) for the calculation of the classification score based on the captured images Ia generated after the target start point for each start point. Here, the "predetermined time interval" is, for instance, an interval which is an integer multiple of the frame period, and is specifically set to a time interval (that is, a time interval in which real-time process can be performed) according to the process capability or the like of the image processing device 1.

In each thread, the calculation of the classification score is continuously performed until either the first threshold value Th1 or the second threshold value Th2 is reached. As a result, the classification scores with different start points are calculated in parallel. For instance, there is a thread with time t11 as the start point for the time t12 and a thread with the time t10 as the start point (see the graph G10) and a thread with start time t11 as the start point (see the graph G11).

Then, in a vicinity of a time t15 in which the lesion site is the target, a parallel process is performed between a thread in which the start point is the time t13 (see the graph G13), a thread in which the start point is the time t14 (see the graph G14), and a thread in which the start point is the time t15 (see the graph G15). Then, in the thread where the start point is the time t13, the first threshold value Th1 is not reached around the period from time t14 to time t16 when the lesion site is the target, due to the fact that the classification score approaches the second threshold value Th2 from the time t13 to the time t14. On the other hand, for the threads started up at time 114 and t15, the classification score reaches the first threshold value Th1 between time t15 and time t16 because the above factor does not exist.

As described above, according to the second manner, by calculating the classification score by starting at the predetermined time interval, it is possible for the score calculation unit 31 to calculate the classification so that the classification score appropriately reaches the first threshold value Th1 when the lesion site is the target. Accordingly, it is possible for the classification unit 34 to appropriately classify a group of the captured images Ia in which the lesion site is the target as a group of images in which the lesion site is present.

(4) Display Example

Next, the display control of the display device 2 to be executed by the display control unit 35 will be described.

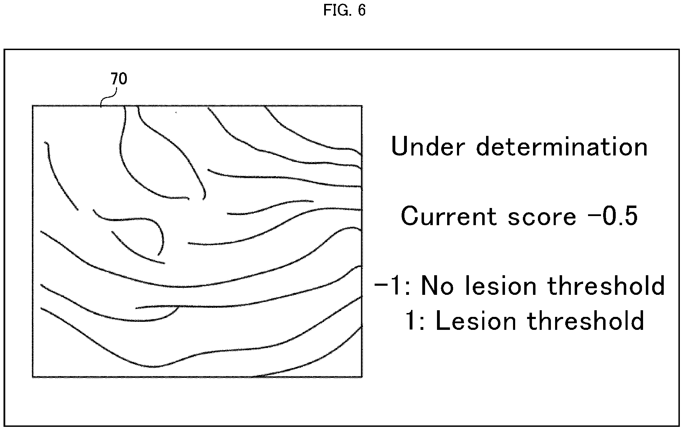

FIG. 6 illustrates a first display example of a display screen displayed by the display device 2 in the endoscopic examination. The display control unit 35 of the image processing device 1 outputs the display information Ib generated based on the captured images Ia acquired by the captured image acquisition unit 30, the classification result generated by the classification unit 34, and the like to the display device 2. The display control unit 35 sends the captured images Ia and the display information Ib to the display device 2, and displays the display screen depicted in FIG. 6 on the display device 2.

In the first display example, the display control unit 35 of the image processing device 1 is provided with a latest image display area 70 representing the latest captured images Ia captured by the captured image acquisition unit 30, and displays information concerning the lesion site on the display screen. Here, the score calculation unit 31 calculates the classification score based on the first manner, and the most recent classification score calculated by the score calculation unit 31 does not reach any threshold value of the first threshold value Th1 and the second threshold value Th2. As a result, the classification unit 34 has not been able to determine the classification. Accordingly, the display control unit 35 recognizes that the classification unit 34 is not able to determine the classification, based on the notification from the classification score or the classification unit 34, and displays, on the display screen, that the classification concerning the presence or absence of the lesion site is undetermined (that is, the presence or absence of a lesion site is being determined). Moreover, the display control unit 35 displays the explanation of the latest classification score (-0.5 in this case) and the threshold value at a current time on the display screen in addition to the information described above.

As described above, in the first display example, in a case where the classification for the captured images Ia is undetermined, the display control unit 35 outputs information indicating that the classification is undetermined together with the information concerning the classification score or the like. Accordingly, it is possible for the display control unit 35 to suitably visualize a current state concerning an existence determination of the lesion site.

Note that the display control unit 35 may display the graph depicted in FIG. 4 on the display screen along with or instead of the display of the classification score. Moreover, while the score calculation unit 31 is calculating the classification score based on the second manner, the display control unit 35 may display respective classification scores corresponding to running threads, may display a classification score closest to either the first threshold Th1 or the second threshold Th2, or may display the graph shown in FIG. 5 on the display screen. Moreover, in a case where the classification for the captured images Ia has not yet been determined instead of or in addition to the display control depicted in the first display example, the display control unit 35 may instruct the sound output unit 16 to output a voice guidance or a predetermined warning sound to inform a user that the classification for the captured images Ia is undetermined. Thereby, it is also possible for the display control unit 35 to suitably notify the examiner know that the classification has not yet been determined.

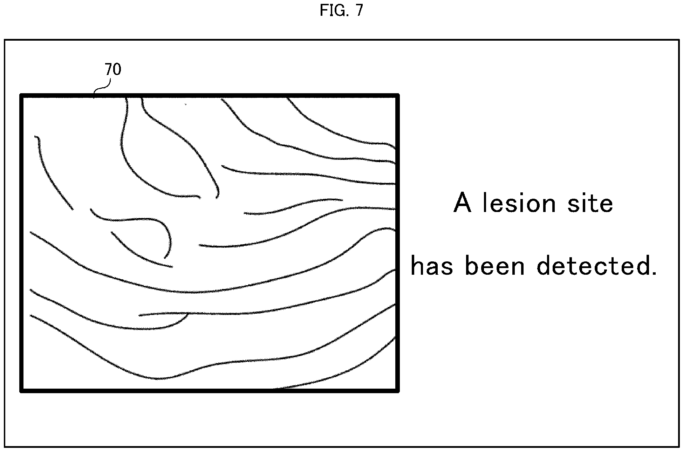

FIG. 7 illustrates a second display example of a display screen displayed by the display device 2 in the endoscopic examination. In the second display example, the classification unit 34 determines that the classification score supplied from the score calculation unit 31 reaches the first threshold value Th1 or more, and supplies the classification result to the display control unit 35 indicating a class in which the lesion site exists. Accordingly, in this case, in order to notify the examiner that the lesion site exists, the display control unit 35 highlights the latest image display area 70 with a border effect and displays a text sentence on the display screen to indicate that the lesion site has been detected.

As described above, in the second display example, in a case where the captured images Ia are classified as having the lesion site, the display control unit 35 outputs information indicating that the lesion site exists (in FIG. 7, the border effect and the text sentence of the latest image display area 70). Accordingly, it is possible for the display control unit 35 to suitably notify the examiner that the lesion site is detected.

Moreover, in a case where the classification score supplied from the score calculation unit 31 is equal to or less than the second threshold value Th2, the display control unit 35 notifies the examiner that the lesion site does not exist. Accordingly, it is possible for the display control unit 35 to suitably prompt the examiner to move a part to be captured by the endoscopic scope 3 (to advance an operation of the endoscopic scope 3).

Note that, instead of the display control depicted in the second display example or in addition to this display control, when the classified score reaches the first threshold value Th1 or more, the display control unit 35 may instruct the sound output unit 16 to output the voice guidance or the predetermined warning sound notifying that the lesion site exists. Also, it is possible for the display control unit 35 to suitably assist the examiner understand the presence of the lesion site. Similarly, the display control unit 35 may instruct the sound output unit 16 to output the voice sound or the warning sound for notifying the examiner that the lesion site does not exist when the classification score reaches the second threshold value Th2 or lower.

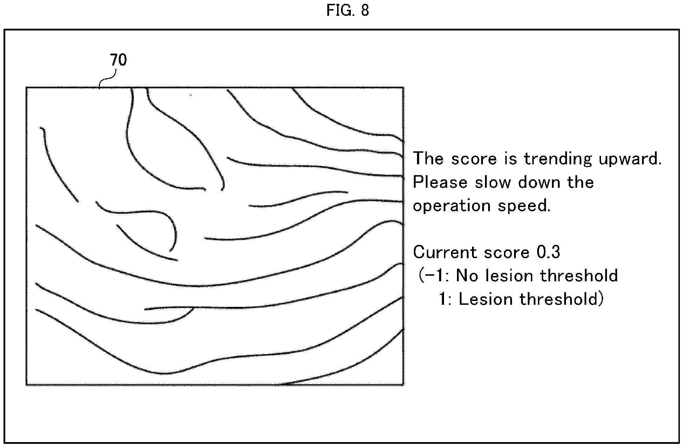

FIG. 8 illustrates a third display example of a display screen displayed by the display device 2 in the endoscopic examination. In the third display example, the display control unit 35 monitors the transition of the classification score by referring to the second calculation information storage unit D2, and determines that the classification score is trending upward. Therefore, the display control unit 35 displays on the display screen that the classification score is trending upward and that an operation speed of the endoscopic scope 3 is slowing down (that is, the target to be captured is fixed as much as possible), along with a current classification score. In this case, for instance, the display control unit 35 determines that the classification score is increasing, when all of a predetermined number of the latest calculated classification scores are a predetermined percentage or higher than the classification scores calculated at a time of an immediately preceding process, or when an average of the predetermined number of the latest calculated classification scores is higher by a predetermined percentage than an average of the predetermined number of the classification scores calculated immediately before the latest calculated classification scores. Note that the determination of the increase of the classification score is not limited to the listed examples, but the increase of the classification score may be determined by any statistical method. As described above, the display control unit 35 may output information concerning a change of the classification score. Moreover, the display control unit 35 may output the operation instruction of the endoscopic scope 3 based on the information concerning the change of the classification score.

As described above, according to the third display example, the display control unit 35 outputs information indicating that the classification score is trending upward in a case where the classification score is trending upward. Accordingly, the display control unit 35 notifies the examiner that the classification score is increasing, and it is possible to suitably suppress that the lesion site is not detected due to the change of a portion captured in the middle of the determination regarding the presence or absence of the lesion site. Note that instead of the display control illustrated in the third display example or in addition to this display control, the display control unit 35 may instruct the sound output unit 16 to output the voice sound or the warning sound for notifying the examiner that the classification score is up to the sound output unit 16.

(5) Process Flow

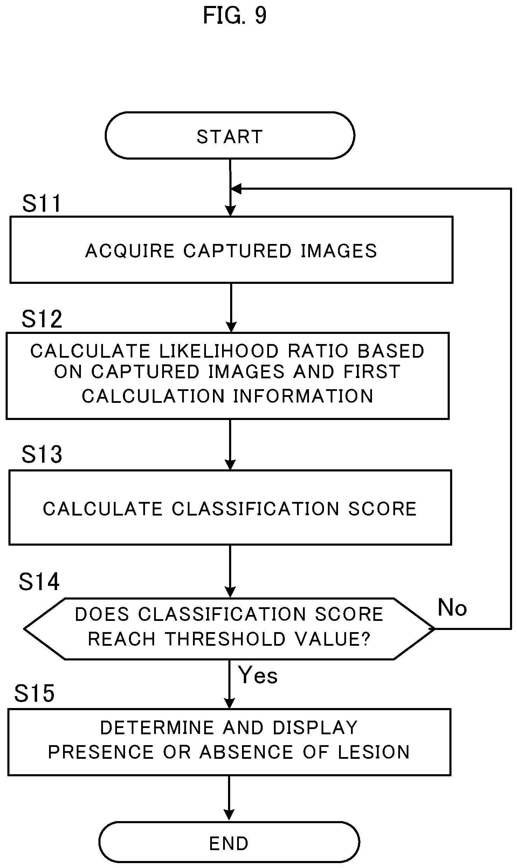

FIG. 9 illustrates an example of a flowchart performed by the image processing device 1, regarding the detection of the lesion site based on the first manner. The image processing device 1 repeatedly executes a process of the flowchart illustrated in FIG. 9 until the endoscopic examination ends. Note that for instance, when detecting a predetermined input or the like to the input unit 14 or the operation unit 36, the image processing device 1 determines that the endoscopic examination is completed.

First, the captured image acquisition unit 30 of the image processing device 1 acquires the captured images Ia (step S11). In this instance, the captured image acquisition unit 30 of the image processing device 1 receives the captured images Ia from the endoscopic scope 3 via the interface 13. Moreover, the display control unit 35 executes a process for displaying the captured images Ia acquired in step S11 on the display device 2.

Next, the image processing device 1 calculates a likelihood ratio based on the captured images Ia acquired in step S12 and the first calculation information stored in the first calculation information storage unit D1 in accordance with the process in this flowchart (step S12). In this instance, the score calculation unit 31 of the image processing device 1 acquires, as the first calculation information, the captured images Ia or the features or the like that have been previously acquired in accordance with the flowchart, and calculates the likelihood ratio based on the first calculation information and the captured image Ia at a current process time acquired in step S12. Moreover, the score calculation unit 31 stores, as the first calculation information, the calculated likelihood ratio, the captured images Ia acquired in step S12 or the features thereof, and the like in the first calculation information storage unit D1.

Next, the image processing device 1 calculates the classification score based on the likelihood ratio or the like calculated in step S12 (step S13). In this case, the score calculation unit 31 calculates an integrated likelihood ratio based on equation (1) by referring to the likelihood ratio or the like stored in the first calculation information storage unit D1, and defines, as the classification score, the calculated integrated likelihood ratio or a function in which the integrated likelihood ratio is used as a variable. Moreover, the score calculation unit 31 stores the calculated classification score and the like as the second calculation information in the second calculation information storage unit D2. Furthermore, the display control unit 35 may perform a process for displaying the classification score and the like calculated by the score calculation unit 31 on the display device 2.

After that, the classification unit 34 of the image processing device 1 determines whether or not the classification score reaches either the first threshold value Th1 or the second threshold value Th2 (step S14). Then, when the classification score is determined to have reached any of the threshold values (step S14; Yes), the classification unit 34 determines (classifies) whether or not the lesion site exists. Furthermore, the display control unit 35 causes the classification unit 34 to display, on the display device 2, information based on the determination result (classification result) indicating the presence or absence of the lesion site by the classification unit 34 (step S15). On the other hand, when determining that the classification score does not reach any of the first threshold value Th1 and the second threshold value Th2 (step S14; No), the classification unit 34 returns the process to step S11. In this instance, the image processing device 1 updates the classification score by further considering the captured image Ia to be acquired at a next process time.

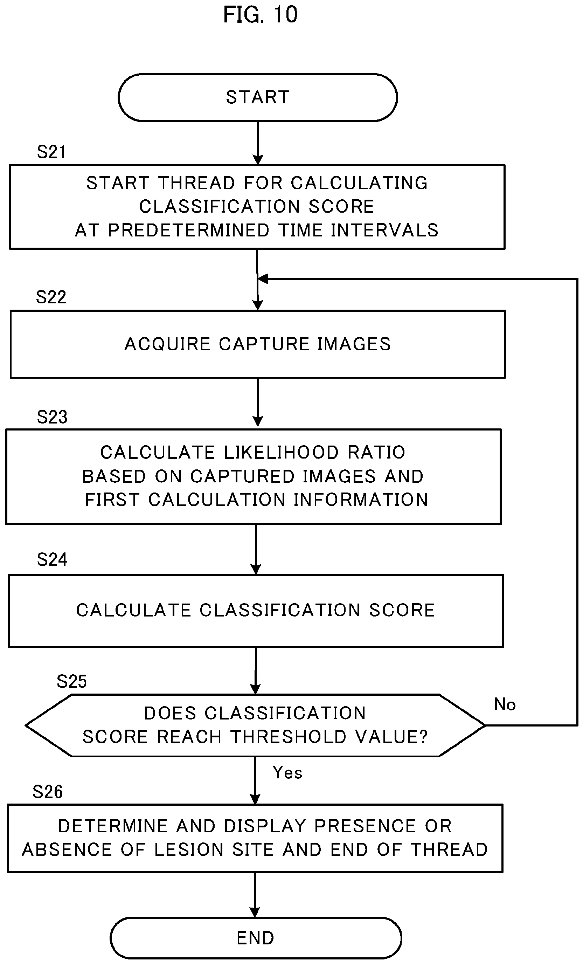

FIG. 10 illustrates an example of a flowchart executed by the image processing device 1 regarding the detection of the lesion site based on the second manner. The image processing device 1 starts the process of the flowchart illustrated in FIG. 10 at every predetermined time interval until the end of the endoscopic examination.

First, the image processing device 1 starts a thread of the classification score calculation at predetermined time intervals (step S21). Next, in each started thread, the image processing device 1 executes the same processes as those in step S11 to step S13 in FIG. 9 in step S22 to step S24. That is, the image processing device 1 performs the acquisition of the captured images Ia (step S22), the calculation of the likelihood ratio (step S23), and the calculation of the classification score (step S24).

Next, the classification unit 34 of the image processing device 1 determines whether or not the classification score reaches either the first threshold value Th1 or the second threshold value Th2 (step S25). When the classification score reaches either one of the threshold values (step S25; Yes), the image processing device 1 determines (classifies) whether or not the lesion site exists, causes the display device 2 to display information based on the determination result (classification result) indicating the presence or absence of the lesion site by the classification unit 34, and terminates the started thread in the flowchart (step S26). On the other hand, when the classification score does not reach either the first threshold value Th1 or the second threshold value Th2 (step S25; No), the image processing device 1 returns the process to step S22. In this instance, in the current thread, the image processing device 1 updates the classification score by further considering the captured images Ia to be acquired at the next process time.

(6) Modifications

Next, a modification suitable for the above-described example embodiment will be described. The following modification may be applied to the example embodiment described above in combination.

(Modification 1)

The image processing device 1 may process a video formed by the captured images Ia generated at the time of the endoscopic examination, after the examination.

For instance, in a case where the video to be processed is designated based on a user input by the input unit 14 at any timing after the examination, the image processing device 1 repeatedly performs a process of the flowchart illustrated in FIG. 9 or FIG. 10 for the captured images Ia of the time series forming the video until it is determined that the video ends being processed.

(Modification 2)

The target to be detected by a detection model is not limited to the lesion site, but may be any point of interest where the examiner needs to focus on. Such spots of interest are not limited to the lesion site, but may also include areas of inflammation, areas where surgical scars or other cuts have occurred, areas where folds or protrusions occur, and areas where the tip portion 38 of the endoscopic scope 3 is easily contacted (jammed) on a wall of a lumen.

(Modification 3)

The classification unit 34 may determine three or more types of classifications, instead of determining a binary classification for the presence or absence of the lesion site. For instance, in this case, the classification unit 34 classifies X+1 types of from an "image group including a lesion type 1" to an "image group including a lesion type X" ("X" is an integer of 2 or more) and of an "image group including no lesion site".

In this case, for instance, in a case where the number of classes to be classified is M, the score calculation unit 31 calculates the integrated likelihood ratio between a kth class and all classes other than the kth class, among the M classes. In this case, for instance, the score calculation unit 31 calculates the integrated likelihood ratio by replacing denominators of a first term and a second term on a right side of the equation (1) with a maximum likelihood in all classes other than the kth class. In this case, instead of the maximum likelihood, the score calculation unit 31 may calculate the integrated likelihood ratio using a sum of the likelihoods of all classes other than the kth class.

Next, based on the classification result by the classification unit 34, the display control unit 35 displays information concerning the presence or absence of the lesion site, and concerning the lesion type in a case where the lesion site is present, on the display device 2. Accordingly, it is possible for the image processing device 1 to suitably assist the examiner in diagnosis.

<Second Example Embodiment>

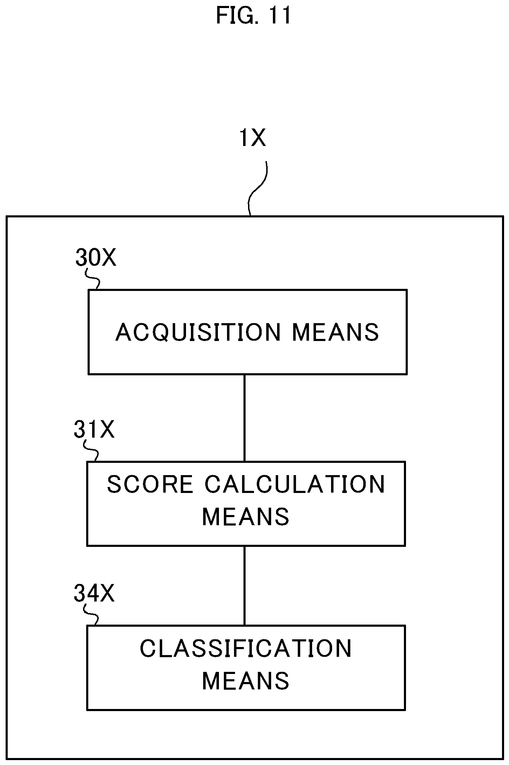

FIG. 11 is a block diagram of an image processing device 1X according to a second example embodiment. The image processing device 1X includes an acquisition means 30X, a score calculation means 31X, and a classification means 34X. The image processing device 1X may be formed by a plurality of devices.

The acquisition means 30X acquires captured images obtained by imaging an examination target by an imaging unit provided on the endoscope. In this instance, the acquisition means 30X may immediately acquire the captured image generated by the imaging unit, or may acquire the captured images which are previously generated by the imaging unit in advance and stored in the storage device, at a predetermined timing. For instance, the acquisition means 30X can be the captured image acquisition means 30 in the first example embodiment (including modifications, and the same applies hereinafter).

The score calculation means 31X calculates a score regarding likelihood in which a point of interest exists in the captured image based on a plurality of captured images in the time series. The score calculation means 31X may be, for instance, the score calculation unit 31 in the first example embodiment.

The classification means 34X classifies a plurality of captured images based on the score. The classification means 34X may be, for instance, the classification unit 34 in the first example embodiment.

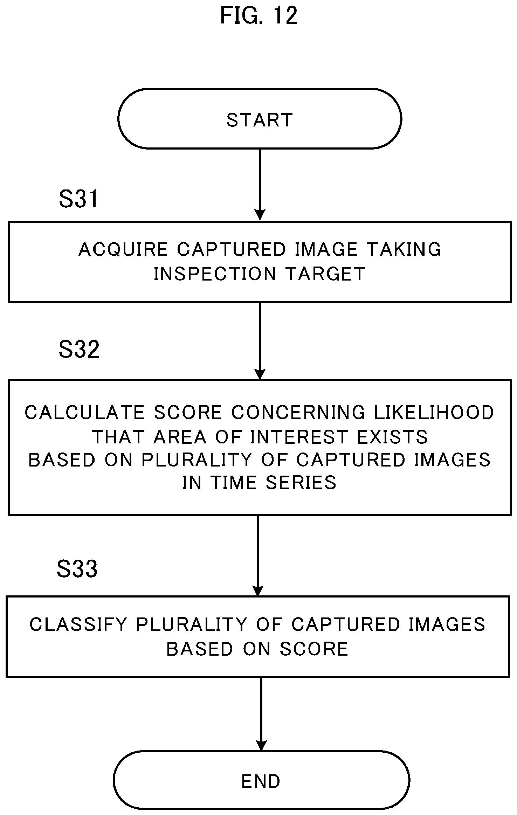

FIG. 12 illustrates an example of a flowchart for explaining a process procedure in the second example embodiment. First, the acquisition means 30X acquires the captured images taken of the examination target by the imaging unit provided on the endoscope (step S31). The score calculation means 31X calculates the score concerning the likelihood of the existence of the point of interest to be focused on in captured images based on a plurality of captured images which are in the time series (step S32). The classification means 34X classifies the plurality of captured images based on the score (step S33).

According to the second example embodiment, it is possible for the image processing device 1X to calculate the score concerning the likelihood of the existence of the point of interest to be focused on based on the plurality of captured images which are in the time series, and to accurately detect the point of interest.

In the example embodiments described above, the program is stored by any type of a non-transitory computer-readable storage medium (non-transitory computer readable storage medium) and can be supplied to a processor or the like that is a computer. The non-transitory computer-readable storage medium include any type of a tangible storage medium. Examples of the non-transitory computer readable storage medium include a magnetic storage medium (that is, a flexible disk, a magnetic tape, a hard disk drive), a magnetic-optical storage medium (that is, a magnetic optical disk), a CD-ROM (Read Only Memory), a CD-R, a CD-R/W, a solid-state memory (that is, a mask ROM, a PROM (Programmable ROM), an EPROM (Erasable PROM), a flash ROM, a RAM (Random Access Memory)). The program may also be provided to the computer by any type of a transitory computer readable storage medium. Examples of the transitory computer readable storage medium include an electrical signal, an optical signal, and an electromagnetic wave. The transitory computer readable storage medium can provide the program to the computer through a wired channel such as wires and optical fibers or a wireless channel.

In addition, some or all of the above example embodiments (including modifications, and the same applies hereinafter) may also be described as in the following supplementary notes, but are not limited to the following.

(Supplementary note 1)

An image processing device comprising:

an acquisition means configured to acquire each captured image which captures an examination target by an imaging unit provided on an endoscope;
a score calculation means configured to calculate a score concerning a likelihood of an existence of a point of interest in captured images based on a plurality of captured images which are in a time series; and
a classification means configured to classify the plurality of captured images based on the score.

(Supplementary note 2)

The image processing device according to supplementary note 1, wherein

the score calculation means calculates the score based on a likelihood ratio concerning a presence or absence of the point of interest in the plurality of captured images, and
the classification means performs a classification concerning the presence or absence of the point of interest in the plurality of captured images.

(Supplementary note 3)

The image processing device according to supplementary note 1 or 2, wherein

the score calculation means updates the score based on each captured image which the acquisition means acquires and the captured images used to calculate the score until the score reaches a threshold value, and
the classification means determines the classification when the score reaches the threshold value.

(Supplementary note 4)

The image processing device according to supplementary note 1, wherein when the score reaches the threshold value, the score calculation means calculates the score based on captured images which the acquisition means acquires after the score reaches the threshold value.

(Supplementary note 5)

The image processing device according to any one of supplementary notes 1 to 3, wherein the score calculation means sets a start point of captured images to be used for calculating the score at predetermined time intervals, and calculates, for each start point being set, the score based on the captured images which the acquisition means acquires after the start point.

(Supplementary note 6)

The image processing device according to any one of supplementary notes 1 to 5, further comprising an output control means configured to output the score and information concerning a classification result by the classification means, by a display device or a sound output device.

(Supplementary note 7)

The image processing device according to supplementary note 6, wherein when the plurality of captured images are classified in response to the existence of the point of interest, the output control means outputs information indicating that the point of interest exists, by the display device or the sound output device.

(Supplementary note 8)

The image processing device according to any one of supplementary notes 6 to 7, wherein when the classification by the classification means has not been determined, the output control means outputs information indicating that the classification has not been determined, by the display device or the sound output device.

(Supplementary note 9)

The image processing device according to any one of supplementary notes 6 to 8, wherein when the score tends to increase, the output control means outputs information indicating that the score tends to increase, by the display device or the sound output device.

(Supplementary note 10)

An image processing method performed by a computer, the image processing method comprising:

acquiring each captured image which captures an examination target by an imaging unit provided on an endoscope;
calculating a score concerning a likelihood of an existence of a point of interest in captured images based on a plurality of captured images which are in a time series; and
classifying the plurality of captured images based on the score.

(Supplementary note 11)

A storage medium storing a program, the program causing a computer to perform a process comprising:

acquiring each captured image which captures an examination target by an imaging unit provided on an endoscope;
calculating a score concerning a likelihood of an existence of a point of interest in captured images based on a plurality of captured images which are in a time series; and
classifying the plurality of captured images based on the score.

While the disclosure has been described with reference to the example embodiments and examples, the disclosure is not limited to the above example embodiments and examples. It will be understood by those of ordinary skill in the art that various changes in form and details may be made therein without departing from the spirit and scope of the present disclosure as defined by the claims.

DESCRIPTION OF SYMBOLS

| | |
|---|---|
| 1, 1X | Image processing device |
| 2 | Display device |
| 3 | Endoscope |
| 4 | Server device |
| 11 | Processor |
| 12 | Memory |
| 13 | Interface |
| 14 | Input unit |
| 15 | Light source unit |
| 16 | Sound output unit |
| 100 | Endoscopy system |

**Claims**

1.  An image processing device comprising:

an acquisition means configured to acquire each captured image which captures an examination target by an imaging unit provided on an endoscope;
a score calculation means configured to calculate a score concerning a likelihood of an existence of a point of interest in captured images based on a plurality of captured images which are in a time series; and
a classification means configured to classify the plurality of captured images based on the score.

2.  The image processing device according to claim 1, wherein the score calculation means calculates the score based on a likelihood ratio concerning a presence or absence of the point of interest in the plurality of captured images, and

the classification means performs a classification concerning the presence or absence of the point of interest in the plurality of captured images.

3. The image processing device according to claim 1 or 2, wherein the score calculation means updates the score based on each captured image which the acquisition means acquires and the captured images used to calculate the score until the score reaches a threshold value, and
the classification means determines the classification when the score reaches the threshold value.

4. The image processing device according to claim 1, wherein when the score reaches the threshold value, the score calculation means calculates the score based on captured images which the acquisition means acquires after the score reaches the threshold value.

5. The image processing device according to any one of claims 1 to 3, wherein the score calculation means sets a start point of captured images to be used for calculating the score at predetermined time intervals, and calculates, for each start point being set, the score based on the captured images which the acquisition means acquires after the start point.

6. The image processing device according to any one of claims 1 to 5, further comprising an output control means configured to output the score and information concerning a classification result by the classification means, by a display device or a sound output device.

7. The image processing device according to claim 6, wherein when the plurality of captured images are classified in response to the existence of the point of interest, the output control means outputs information indicating that the point of interest exists, by the display device or the sound output device.

8. The image processing device according to claim 6 or 7, wherein when the classification by the classification means has not been determined, the output control means outputs information indicating that the classification has not been determined, by the display device or the sound output device.

9. The image processing device according to any one of claims 6 to 8, wherein when the score tends to increase, the output control means outputs information indicating that the score tends to increase, by the display device or the sound output device.

10. An image processing method performed by a computer, the image processing method comprising:

   acquiring each captured image which captures an examination target by an imaging unit provided on an endoscope;
   calculating a score concerning a likelihood of an existence of a point of interest in captured images based on a plurality of captured images which are in a time series; and
   classifying the plurality of captured images based on the score.

11. A storage medium storing a program, the program causing a computer to perform a process comprising:

   acquiring each captured image which captures an examination target by an imaging unit provided on an endoscope;
   calculating a score concerning a likelihood of an existence of a point of interest in captured images based on a plurality of captured images which are in a time series; and
   classifying the plurality of captured images based on the score.

EP 4 327 721 A1

# FIG. 1

## 100: ENDOSCOPY SYSTEM

15

FIG. 2

FIG. 3

FIG. 4

FIG. 5

FIG. 6

Under determination

Current score −0.5

−1: No lesion threshold
1: Lesion threshold

FIG. 7

A lesion site

has been detected.

FIG. 8

70

The score is trending upward.
Please slow down the
operation speed.

Current score 0.3
(−1: No lesion threshold
1: Lesion threshold)

# FIG. 9

START

S11
ACQUIRE CAPTURED IMAGES

S12
CALCULATE LIKELIHOOD RATIO BASED ON CAPTURED IMAGES AND FIRST CALCULATION INFORMATION

S13
CALCULATE CLASSIFICATION SCORE

S14
DOES CLASSIFICATION SCORE REACH THRESHOLD VALUE?  No

Yes

S15
DETERMINE AND DISPLAY PRESENCE OR ABSENCE OF LESION

END

## FIG. 10

START

S21
START THREAD FOR CALCULATING
CLASSIFICATION SCORE
AT PREDETERMINED TIME INTERVALS

S22
ACQUIRE CAPTURE IMAGES

S23
CALCULATE LIKELIHOOD RATIO
BASED ON CAPTURED IMAGES AND
FIRST CALCULATION INFORMATION

S24
CALCULATE CLASSIFICATION SCORE

S25
DOES CLASSIFICATION
SCORE REACH THRESHOLD VALUE?    No

Yes

S26
DETERMINE AND DISPLAY PRESENCE OR
ABSENCE OF LESION SITE AND END OF THREAD

END

FIG. 11

1X

30X

ACQUISITION MEANS

31X

SCORE CALCULATION
MEANS

34X

CLASSIFICATION
MEANS

# FIG. 12

START

S31

ACQUIRE CAPTURED IMAGE TAKING
INSPECTION TARGET

S32

CALCULATE SCORE CONCERNING LIKELIHOOD
THAT AREA OF INTEREST EXISTS
BASED ON PLURALITY OF CAPTURED IMAGES
IN TIME SERIES

S33

CLASSIFY PLURALITY OF CAPTURED IMAGES
BASED ON SCORE

END

**EP 4 327 721 A1**

## INTERNATIONAL SEARCH REPORT

International application No.

**PCT/JP2021/016473**

### A. CLASSIFICATION OF SUBJECT MATTER

*A61B 1/045*(2006.01)i
FI: A61B1/045 618; A61B1/045 614

According to International Patent Classification (IPC) or to both national classification and IPC

### B. FIELDS SEARCHED

Minimum documentation searched (classification system followed by classification symbols)

A61B1/00-1/32

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2016/110993 A1 (OLYMPUS CORP.) 14 July 2016 (2016-07-14) paragraphs [0026]-[0092], fig. 1-11 | 1, 5-7, 10-11 |
| Y | | 2-4, 8-9 |
| X | WO 2020/170791 A1 (FUJIFILM CORP.) 27 August 2020 (2020-08-27) paragraphs [0078]-[0082], fig. 1, 2 | 1, 6-7, 10-11 |
| Y | | 2-5, 8-9 |
| Y | WO 2020/194497 A1 (NEC CORP.) 01 October 2020 (2020-10-01) paragraphs [0029]-[0060], fig. 1-3 | 2-5, 8-9 |
| A | JP 2019-180966 A (SHOWA UNIVERSITY) 24 October 2019 (2019-10-24) paragraphs [0004], [0028], [0069] | 1-11 |
| A | WO 2020/165978 A1 (OLYMPUS CORP.) 20 August 2020 (2020-08-20) paragraph [0041] | 1-11 |
| A | WO 2019/138772 A1 (FUJIFILM CORP.) 18 July 2019 (2019-07-18) paragraphs [0084]-[0189], fig. 1-9 | 1-11 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 June 2021** | **06 July 2021** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

27

**INTERNATIONAL SEARCH REPORT**
Information on patent family members

International application No.

**PCT/JP2021/016473**

| Patent document cited in search report | | | Publication date (day/month/year) | Patent family member(s) | | | Publication date (day/month/year) |
|---|---|---|---|---|---|---|---|
| WO | 2016/110993 | A1 | 14 July 2016 | US 2017/0296043 A1 paragraphs [0069]-[0136], fig. 1-11 | | | |
| WO | 2020/170791 | A1 | 27 August 2020 | (Family: none) | | | |
| WO | 2020/194497 | A1 | 01 October 2020 | (Family: none) | | | |
| JP | 2019-180966 | A | 24 October 2019 | WO 2019/198808 A1 | | | |
| WO | 2020/165978 | A1 | 20 August 2020 | (Family: none) | | | |
| WO | 2019/138772 | A1 | 18 July 2019 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020003607 A

- WO 2020194497 A